Europäisches Patentamt

European Patent Office

Office européen des brevets

(11) Publication number : **0 685 586 A2**

(12) **EUROPEAN PATENT APPLICATION**

(21) Application number : **95303579.7**

(22) Date of filing : **26.05.95**

(51) Int. Cl.<sup>6</sup> : **D04H 13/00**

(30) Priority : **26.05.94 US 249416**

(43) Date of publication of application :
**06.12.95 Bulletin 95/49**

(84) Designated Contracting States :
**DE ES FR GB IT NL SE**

(71) Applicant : **INTERNATIONAL PAPER COMPANY**
**2 Manhattanville Road**
**Purchase New York 10577 (US)**

(72) Inventor : **Coslett, W. Andrew**
**412 Main Street**
**Medfield, Massachusetts 02052 (US)**
Inventor : **Leonguerrero, Leonardo B.**
**119 Mill Street**
**Franklin, Massachusetts 02038 (US)**
Inventor : **Srinivasan, Ramesh**
**3 Kenmar Drive, Apt. 11**
**Billerica, Massachusetts 01821 (US)**
Inventor : **Gardner, Daniel D.**
**1815 Marian Marisville Road**
**Marion, Ohio 43302 (US)**

(74) Representative : **Kearney, Kevin David Nicholas et al**
**KILBURN & STRODE**
**30 John Street**
**London, WC1N 2DD (GB)**

(54) **Unidirectionally stretchable elastomeric trilaminate and method for its manufacture.**

(57) An elastomeric trilaminate fabric for use in the waist band or leg cuff of disposable diapers, training pants and other similar products. The trilaminate has a middle elastomeric web sandwiched between a pair of gatherable inelastic webs. The elastomeric web may take the form of a scrim or a film made from elastomeric material. The nonwoven webs are made from staple or bicomponent fibers which have been carded and thermal bonded or spunbond. During manufacture, the nonwoven webs are ultrasonically pattern bonded to each other while the elastomeric web is in a stretched state. The elastomeric web is sandwiched between and captured by the bonded gatherable webs. Upon release of the tension applied to the elastomeric web, it will relax to an unstretched state. As the elastomeric web contracts, the nonwoven webs gather between the bonding points. The result is a stretchable laminate which elastically returns to an unstretched state when tension is removed.

EP 0 685 586 A2

### Field of the Invention

This invention generally relates to elastomeric laminates comprising nonwoven fabric. In particular, the invention relates to elastomeric laminates in which nonwoven gatherable webs are laminated on both sides of a layer of elastomeric material.

### Background of the Invention

Stretchable composites made of elastomeric material laminated to a nonwoven web are ideally suited for use in consumer disposable articles such as diapers, adult incontinence pads and sanitary napkins. Such composites conform to the shape of the human body and thus provide an excellent fit for the wearer.

One such composite material is disclosed in U.S. Patent No. 4,720,415 issued to Vander Wielen et al. and numerous other patents assigned to Kimberly-Clark Corporation. The Vander Wielen patent discloses a composite elastic material comprising an elastic web, i.e., a nonwoven web of meltblown elastomeric fibers, bonded to at least one gatherable web, i.e., a spunbonded polyester fiber material. The composite material is produced by stretching the elastic web to elongate it and bonding the elongated web to at least one gatherable web under conditions which soften at least a portion of the elastic web to form the bonded composite web of elastic material. Upon relaxation of the composite material, the elastic web contracts to form gathers in the gatherable web. The bonding may be effectuated by pattern embossing overlaid elastic and gatherable webs with at least portions of the elastic web being heated to its softening temperature, e.g., using thermal bonding or ultrasonic welding. The disclosure further states that elastic films can be used in place of nonwoven fibrous elastic webs.

Another patent of interest and assigned to Kimberly-Clark is U.S. Patent No. 4,657,802 to Morman. The Morman patent discloses a composite nonwoven elastic web including a nonwoven elastic web joined to a nonwoven gathered web on one or both sides. The term "nonwoven web" is defined to include an apertured film. The elastic web is made of meltblown microfibers or apertured film. The gathered webs are made of meltblown or spunbond fibers. Mention is made that the gatherable web can be joined to the elastic web by heat bonding or sonic bonding, but no examples are given. Morman also mentions that the gatherable web may take the form of a carded web deposited on a tacky elastomeric web.

Ultrasonic bonding of thin flexible webs to form an elastic laminate is also disclosed in U.S. Patent No. 4,404,052 issued to Persson et al. and assigned to The Procter & Gamble Company. This patent specifically refers to an elasticized trilaminate consisting of a nonwoven polyester top web, an elastomeric butadiene/styrene copolymer middle web and a polyethylene bottom web.

A bulked composite web comprising a differentially tensioned reticulated web of elastic material bonded to at least one gatherable web is disclosed in U.S. Patent No. 4,606,964 issued to Wideman and assigned to Kimberly-Clark Corporation. The reticulated web is formed by bonding individual strands at crossover points. The individual strands are differentially tensioned prior to bonding. It is disclosed that the reticulated web can be joined to the gatherable web by ultrasonic bonding.

Also of interest is U.S. Patent No. 4,573,991 issued to Pieniak et al. and assigned to Personal Products Company. The Pieniak patent discloses a laminated structure comprising a reticulated elastic member disposed between two substrates of flexible gatherable material. The substrates are bonded together through the apertures in the reticulated elastic member using adhesive.

### Brief Summary of the Invention

The present invention is a composite web formed by sandwiching an elastomeric web between a pair of inelastic webs. In accordance with one preferred embodiment, the elastomeric middle layer is a film made of elastomeric material. In accordance with another preferred embodiment, the elastomeric middle layer is a reticulate net or scrim made of elastomeric material. The scrim stretch may be unidirectional or bidirectional. The outer layers are gatherable webs of inelastic material. The three webs are laminated to form a composite web which is stretchable in only the machine direction or in both the machine and cross directions.

The nonwoven gatherable webs may take the form of spunbonded fibers or carded and thermal-bonded fibers. The fibers are made of non-elastomeric material, such as polypropylene, polyester, polyethylene, polyamide or rayon. Bicomponent fibers, such as polyethylene/polypropylene fibers, can also be used. These bicomponent fibers may be in sheath-core or side-by-side form or any other conventional form. The basis weight range for the nonwoven web can be in the weight range of 12-70 $gm/yd^2$. The fiber finish can be hydrophilic or hydrophobic.

The process for making the above-described nonwoven-elastomeric-nonwoven stretch trilaminate uses ultrasonic bonding to bond the two gatherable webs to the elastomeric layer. The process utilizes suitable bond rolls that allow point bonding of all three layers. The particular pattern employed and the bond area (%) of the roll will influence the final stretch properties of the trilaminate.

The general process of ultrasonic lamination consists of bonding layers of material (nonwoven webs, films, etc.) using an ultrasonic horn and a bond roll (engraved with a chosen geometric pattern). The materials to be laminated (nonwoven, film, etc.) are fed from an unwinding system and led through a series of idlers/rollers that enable control of tension during bonding.

In the disclosed process for manufacturing a machine-direction stretchable laminate using elastomeric scrim, two webs of inelastic material are laminated together with the scrim sandwiched therebetween. The inelastic webs are fed unstretched to a bond roll. The elastomeric scrim is fed to the bond roll (bond area ≈ 0.5-2.0%) after being stretched to a desired extent. This is done by feeding the scrim at a proportionately lower speed (e.g., half the speed at which the gatherable webs are fed) to attain a required stretch (in this case 2X).

The rate at which the elastomeric scrim is fed is one key factor in imparting desired stretch. This is controlled by using a set of driven nip rolls that regulate the speed of the elastomeric scrim before being fed to the bond roll.

All three layers are point-bonded on the bond roll. As soon as the trilaminate exits the bond roll, it is taken up at very low tensions by a winder. Under these conditions, the scrim recovers from its stretching and bounces back to its original length. This now causes the top and bottom layers to bounce back, taking with them a proportionate amount of (in this case two times) material in the form of pleats. The nonwoven-elastomeric-nonwoven trilaminate thus shrinks in the machine direction by a factor of the stretch imparted to the elastomeric scrim.

In a given unit length of the final trilaminate, there exists twice the length of top and bottom layers (in a pleated form) compared to one unit length of scrim. In other words, to obtain a trilaminate that stretches 100% (2X stretch), one unit length of scrim and four unit lengths of top and bottom layers are needed.

In accordance with the invention, a product can be obtained which stretches from zero to greater than or equal to three times its original length and recovers at least 75% of its original length upon being relaxed. Pleating of the different layers results in a very high caliper for the product. The aesthetics of the laminate are thus vastly enhanced by its bulk. The product possesses excellent peel strength between the different layers, is soft and comfortable, and has uniform appearance.

The use of an elastomeric scrim is more advantageous than an elastomeric film because the scrim does not result in any neckdown of the final laminate and thus no width loss is experienced, resulting in a cost advantage. The film constricts in both machine and cross directions upon being stretched in the machine direction. In contrast, in the case of the scrim, elastomeric filaments get drawn in the machine direction only (between the crossover points) when stretched in machine direction. The cross direction remains stable. This reduces overall usage of scrim for every length of trilaminate produced.

## Brief Description of the Drawings

FIG. 1 is a perspective view showing the process concept for manufacturing machine direction stretch fabric using an elastomeric scrim in accordance with the preferred embodiment of the invention.

FIG. 2 is a diagram showing the wire pattern used to ultrasonically bond the layers making up the trilaminate in accordance with the preferred embodiments of the invention.

## Detailed Description of the Preferred Embodiments

The present invention is an elastomeric nonwoven-film-nonwoven (A-B-A) laminate which is ideally suited for use in the waist band or leg cuff of disposable diapers, training pants and other similar products. The nonwoven webs are made from staple or bicomponent fibers which have been carded and thermal bonded or spunbonded. An elastomeric layer is sandwiched between the nonwoven webs and laminated thereto. The elastomeric layer may take the form of a film or a scrim (i.e., network) made of elastomeric material.

The preferred nonwoven web is a 20 $gm/yd^2$ thermal-bonded web composed of 100% carded hydrophobic polypropylene staple fiber having a denier of 2.2 and an average fiber length of about 1.5 inches. Alternatively, the nonwoven webs may comprise 100% polyester, polyethylene or polyamide staple fibers, polypropylene/rayon blends (with up to 60 wt.% rayon) or 100% polyethylene/ polypropylene bicomponent or biconstituent fibers. The fibers may have either a hydrophobic or a hydrophilic finish. The basis weight of each nonwoven can be in the range of 12 to 70 $gm/yd^2$.

In accordance with the method of the invention, the nonwoven webs are ultrasonically pattern bonded to each other, with the elastomeric layer therebetween, using a patterned bonding roll. The preferred geometry of the bonding roll includes a point density of about 27 $points/in.^2$ that fuses 1.5-2.0% of the surface of the material contacting the bond roll. The bonding points may be separated by 0.25 inch in the machine direction and 0.312 inch in the cross direction.

The nonwoven webs are ultrasonically bonded to the elastomeric web while the latter is in a stretched state. Upon release of the tension applied to the elastomeric web, the latter will relax to an unstretched state. As the elastomeric web contracts, the nonwoven webs gather between the bonding points. The result is a stretchable laminate which has a proclivity to return to its unstretched state.

Referring to the drawing, a laminated fabric in accordance with the preferred embodiment of the invention is formed by ultrasonic bonding a scrim 1 made a elastomeric material between two layers 2 and 3 made of inelastic material. Preferably, the inelastic layers are nonwoven webs made from carded and thermal bonded synthetic fibers, such as the above-specified polypropylene staple fibers. Alternatively, the inelastic layers 2 and 3 can take the form of apertured films.

In accordance with the process of the invention, elastomeric scrim 1 and inelastic layers 2 and 3 are unwound from supply rolls as shown in the drawing. The scrim is nipped between two black rubber nip rolls 4 (hereinafter "pre-stretch rolls") which initiate stretch of the scrim.

The scrim and the inelastic layers are then converged at a guide roll 11 so that the inelastic layers sandwich the elastomeric scrim. Thereafter, the three webs ride together on the outer circumferential surface of a rotating sonic bond roll 5, which may comprise an engraved pattern or a wire pattern or a patterned plastic sleeve. The outer circumferential surface of the sonic bond roll thus forms an anvil having a pattern of discrete bonding protuberances protruding radially outward. The ultrasonic bonding will occur at the bonding protuberances, while no bonding will occur over the reliefs separating the bonding protuberances.

A bank of ultrasonic welding units 6 (hereinafter "horns") are positioned directly above sonic bond roll 5. Although the drawing depicts a bank having two horns, any number of horns may arranged in a line depending on the desired width of the trilaminate and the width of each horn. The ultrasonic horns are biased toward the driven sonic bond roll to press the layers to be laminated against the relief-type bonding pattern on the surface of the sonic bond roll 5.

In a conventional manner, the ultrasonic horns are activated to laminate the three webs on-the-fly. Each unit has a transducer which converts electrical vibrations to mechanical vibrations, thereby transmitting the vibrations to the material to be bonded via the booster and horn and toward the opposing bonding pattern on the surface of sonic bond roll 5. Ultrasonic bonding will occur at each spot on the webs overlying a bonding protuberance. In this way, for example, the outer inelastic webs of the trilaminate can be pattern bonded together with the stretched elastomeric scrim trapped between the bonding spots.

In the next stage of the process, the trilaminate fabric passes through a pair of opposing rolls 7a and 7b, which control in-wound stretch of the trilaminate. Roll 7a is a rubber pinch roll and roll 7b is a metal roll.

The trilaminate is optionally spread by a spreader roll 8 to remove wrinkles. Then the trilaminate is wound by a pair of surface winder rolls 9, both of which are driven. The surface winder has slitting capability. The final product is a wound roll 10 of MD stretch trilaminate fabric.

In one example, the peripheral surface speed of pre-stretch rolls 4 was 25.0 fpm and the peripheral surface speed of sonic bond roll 5 was 50.0 fpm. The difference in peripheral surface speeds between the sonic bond roll 5 and the pre-stretch rolls 4 produces a tension which stretches the elastomeric scrim 1. The peripheral surface speed of pinch roll 7a was 50.0 fpm. Thus, the elastomeric scrim is maintained in its stretched condition between the sonic bond roll 5 and the pinch roll 7a. The peripheral surface speed of the winder rolls 9 was 36.0 fpm. The difference in peripheral surface speeds between the pinch roll 7a and the winder rolls 9 releases the tension in the elastomeric scrim 1.

The elastomeric scrim used in the invention may be made from commercially available thermoplastic elastomeric polymers, e.g., poly(styrene)/poly(ethylene-butylene)/poly(styrene) block copolymers. Such scrims may be purchased in a variety of bidirectional meshes, e.g., $5 \times 5$, $7 \times 7$, $15 \times 5$, $18 \times 5$ or $18 \times 14$ mesh, where the first numeral in each mesh size refers to the number of transverse strands per inch measured in the machine direction and the second numeral in each mesh size refers to the number of longitudinal strands per inch measured in the cross direction.

During developmental runs, the best results were obtained using an elastomeric scrim having a $15 \times 5$ mesh, bidirectional stretch and a basis weight of 85.0 gsy. Samples of $15 \times 5$ mesh with bidirectional stretch had the following properties:

TABLE 1

| Properties | 1 | 2 | Sample No. 3 | 4 | 5 | 6 | Avg. |
|---|---|---|---|---|---|---|---|
| Weight, gsy | 86.2 | 88.8 | 73.0 | 70.2 | 98.5 | 98.5 | 85.9 |
| Thickness, mils | 24.7 | 25.3 | 23.2 | 23.5 | 26.4 | 26.7 | 24.9 |
| MD Tensile, lbs. | 1.382 | 1.382 | 1.132 | 1.132 | 1.731 | 1.607 | 1.394 |
| MD Elongation, % | 632.9 | 658.0 | 658.7 | 764.8 | 655.8 | 666.0 | 672.7 |
| MD Load a 5% Elongation, lbs. | 0.195 | 0.158 | 0.162 | - | 0.307 | 0.294 | 0.225 |
| MD Load a 10% Elongation, lbs. | 0.261 | 0.275 | 0.232 | - | 0.307 | 0.294 | 0.275 |
| MD Load a 100% Elongation, lbs. | 0.407 | 0.393 | 0.337 | 0.307 | 0.451 | 0.482 | 0.396 |
| MD Load a 200% Elongation, lbs. | 0.455 | 0.443 | 0.382 | 0.375 | 0.507 | 0.532 | 0.449 |
| CD Tensile, lbs. | 0.753 | 0.803 | 0.706 | 0.702 | 0.932 | 0.961 | 0.809 |
| CD Elongation, % | 660.1 | 651.7 | 657.0 | 644.5 | 658.2 | 654.6 | 654.4 |
| CD Load a 5% Elongation, lbs. | 0.048 | 0.048 | 0.047 | 0.054 | 0.069 | 0.073 | 0.056 |
| CD Load a 10% Elongation, lbs. | 0.086 | 0.085 | 0.082 | 0.099 | 0.126 | 0.127 | 0.101 |
| CD Load a 100% Elongation, lbs. | 0.186 | 0.188 | 0.167 | 0.189 | 0.252 | 0.258 | 0.207 |
| CD Load a 200% Elongation, lbs. | 0.244 | 0.256 | 0.215 | 0.228 | 0.308 | 0.318 | 0.261 |
| MD Recovery, % | 25.0 | - | 10.0 | 17.5 | - | 11.3 | 8.70 |
| CD Recovery, % | 19.1 | 24.4 | 27.1 | 26.3 | 17.7 | 25.5 | 21.5 |

During these runs, the 15 × 5 scrim was fed at 23.5 fpm, while two T190 polypropylene thermal-bonded nonwoven webs, each having a basis weight of 18 gsy, were fed at 53.0 fpm. The unwinding tension for the scrim was 100.0 gm, while the unwinding tension for each nonwoven web was 175.0 gm. The surface of the black rubber nip (stretch) roll 4 traveled at 25.0 fpm. The tension of scrim 1 between nip roll 4 and sonic bond roll 5 was 225.0 gm. The sonic bond roll had a wire pattern (e.g., # WP 0008), which traveled at a speed of 53.0 fpm. The horn pressure was 10.0 psi for each of the left and right horns. Following ultrasonic bonding, the tension of the trilaminate between sonic bond roll 5 and pinch roll 7a was 400.0 gm. The surface of pinch roll 7a traveled at 53.0 fpm. The surface of surface winder 9 traveled at 36.0 fpm. The winding tension was 300.0 gm. Using the foregoing process parameters, an elastomeric trilaminate fabric was produced having an available stretch of 100%. The number of breaks in the scrim, due to ultrasonic bonding, was $\leqq$2.0 breaks/in.$^2$.

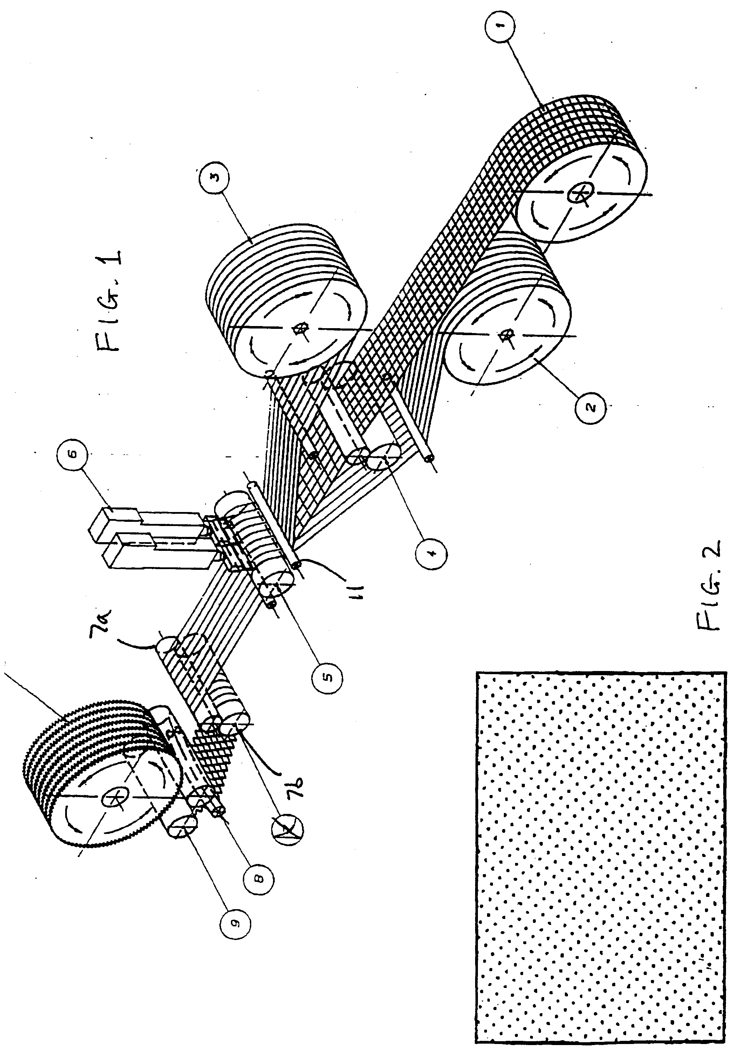

Nonwoven-elastomeric scrim-nonwoven trilaminate fabric was produced using two different pattern rolls: a WP 0008 wire pattern and a WP 00012 wire pattern. The arrangement of wires for these patterns is generally shown in FIG. 2, the WP 0008 and WP 00012 having different dimensions as follows:

TABLE 2

| | Pattern | |
|---|---|---|
| | 0008 | 00012 |
| Bond spots per sq. inch | 27 | 13 |
| MD spot to spot, inch | 0.312 | 0.250 |
| CD spot to spot, inch | 0.250 | 0.500 |
| Spot area, sq. inch | 0.0007392 | 0.0007392 |
| Bond area, % | 1.99 | 0.96 |

For the WP 0008 and WP 00012 wire patterns, the trilaminate had the properties listed in Tables 3 and 4, respectively.

5

TABLE 3

| Properties | Sample No. | | | | |
| | 1 | 2 | 3 | 4 | Avg. |
|---|---|---|---|---|---|
| Basis Weight, gsy | 158.4 | 154.6 | 153.9 | 155.4 | 155.6 |
| Thickness, mils | 102.0 | 102.1 | 105.3 | 104.8 | 103.5 |
| MD Strip Tensile, gm/in. | 3576 | 3262 | 3131 | 3249 | 3304 |
| CD Strip Tensile, gm/in. | 1489 | 1376 | 1639 | 1262 | 1441 |
| MD Elongation, % | 179 | 155 | 161 | 166 | 165 |
| CD Elongation, % | 116 | 90 | 92 | 82 | 95 |
| MD Load a 50% Elongation, lbs. | 120.3 | 126.9 | 124.9 | 119.9 | 123 |
| CD Load a 50% Elongation, lbs. | 826.1 | 942.1 | 1081 | 908.3 | 939.4 |
| MD Peel, gm | 380.3 | 331.4 | 271.1 | 286.5 | 317.3 |
| Available Stretch, % | 112.5 | 112.5 | 112.5 | 112.5 | 112.5 |

TABLE 4

| Properties | Sample No. | | | | |
| | 1 | 2 | 3 | 4 | Avg. |
|---|---|---|---|---|---|
| Basis Weight, gsy | 146.4 | 160.9 | 142.0 | 156.2 | 151.4 |
| Thickness, mils | 98.5 | 95.2 | 123.7 | 88.7 | 101.5 |
| MD Strip Tensile, gm/in. | 3097 | 3138 | 3755 | 3456 | 3361 |
| CD Strip Tensile, gm/in. | 1073 | 973 | 1491 | 1232 | 1192 |
| MD Elongation, % | 151 | 136 | 169 | 152 | 152 |
| CD Elongation, % | 88 | 64 | 112 | 87 | 88 |
| MD Load a 50% Elongation, lbs. | 113.7 | 109.2 | 113.7 | 109.2 | 111.5 |
| CD Load a 50% Elongation, lbs. | 721.2 | 825.7 | 866.5 | 850.9 | 816.1 |
| MD Peel, gm | 170.0 | 150.3 | 135.8 | 166.9 | 156.5 |
| CD Peel, gm | 341.5 | 322.2 | 256.7 | 210.7 | 282.8 |
| Available Stretch, % | 100.0 | 100.0 | 100.0 | 100.0 | 100.0 |

The amount of available stretch in the laminate can be varied by four main factors: (1) type of elastomeric scrim (varying the mesh size, kind of elastomeric blend employed, etc.) ; (2) rate of feed of scrim; (3) bond roll pattern (includes geometry, bond area etc.) ; and (4) method of packing (winding into rolls, spooling, festooning, etc.).

While pleating occurs in the machine direction, no neckdown occurs in the cross direction. The neckdown in the cross direction can be induced by varying the type of scrim employed.

Alternatively, the same apparatus shown in the drawing could be used to make MD stretch trilaminate fabric in which the elastomeric scrim is replaced by an elastomeric film. The two nonwoven webs are bonded to each other by the ultrasonic horns 6 acting against the pattern on the sonic bond roll 5. The film is composed of compounds which melt at a lower temperature than the melting point of the polymeric material used to bond the nonwoven webs together. Thus, the film does not contribute to the bond integrity of the laminate. The preferred geometry of the bond roll is WP 0008 with a point density of 27 points/in.[2] that fuses 1.5-2.0% of the surface of the material contacting the bond roll. The preferred pattern layout is a five-point array with spots 0.312" apart in the machine direction and 0.250" apart in the cross direction. The bonded laminate then passes to the tension isolation zone that permits it to relax (contract) between the bond point and the winder. The amount of stretch in the resulting laminate has been demonstrated to range from 10% to 200%. The permissible range of bond areas is 0.75% to 25%.

During developmental runs, a clear or white elastomeric film made from styrene-butadiene copolymer or other conventional elastomer was fed at 24.5 fpm, while two T190 polypropylene thermal-bonded nonwoven webs, each having a basis weight of 18 gsy, were fed at 52.0 fpm. The unwinding tension for the film was 125.0 gm, while the unwinding tension for each nonwoven web was 100.0 gm. The surface of the black rubber nip (stretch) roll 4 traveled at 25.0 fpm. The tension of scrim 1 between nip roll 4 and sonic bond roll 5 was 550.0 gm. The sonic bond roll had a wire pattern (e.g., # WP 0008), which traveled at a speed of 52.5 fpm. The horn pressure was 10.0 psi for each of the left and right horns. Following ultrasonic bonding, the tension of the trilaminate between sonic bond roll 5 and pinch roll 7a was 300.0 gm. The surface of pinch roll 7a traveled at 53.5 fpm. The surface of surface winder 9 traveled at 43.0 fpm. The winding tension was 200.0 gm. Using the foregoing process parameters, an elastomeric trilaminate fabric was produced having an available stretch of 100%.

The resulting nonwoven-based MD stretch laminate had the following properties: weight - 112.5 gsy ; thickness - 117.0 mils; strip tensile strength: MD - 3050 gm/inch, CD - 1653 gm/inch; elongation: MD - 182%, CD - 110%; load at 50% elongation: MD - 169 gm, CD - 984 gm; peel strength: MD - 112.2 gm, CD - 224.6 gm; MD stretch -116%.

As previously discussed, the nonwoven gatherable webs can be made from fibers different than polypropylene staple fibers, e.g., polyester, polyethylene or polyamide staple fibers, polypropylene/rayon blends (up to 60 wt.% rayon) or 100% polyethylene/polypropylene bicomponent or biconstituent fibers. Alternatively, apertured films made from polyethylene, ethyl vinyl acetate or other suitable polymer can be used in place of nonwoven webs.

The foregoing process and apparatus have been disclosed for the purpose of illustration. Variations and modifications of the disclosed process and apparatus will be readily apparent to practitioners skilled in the arts of ultrasonic bonding and the manufacture of elasticized material. All such variations and modifications which do not depart from the concept of the present invention are intended to be encompassed by the claims set forth hereinafter.

In its broadest aspects the invention extends to a laminated fabric comprising first and second nonwoven webs of inelastic material ultrasonically bonded directly to each other at spaced intervals through holes in an elastomeric scrim sandwiched therebetween.

Each of said first and second nonwoven webs preferably comprises thermally bonded fibres, or spunbond fibres.

The scrim may have a first mesh in a machine direction which is greater than a second mesh in a cross direction.

Alternatively the scrim may have a first mesh in a machine direction which is equal to a second mesh in a cross direction.

As a further possibility the scrim may have a plurality of mutually parallel machine direction strands and a plurality of mutually parallel cross direction strands, said machine direction strands and said cross direction strands being fused at their crossover points.

The scrim may comprise a plurality of interconnected strands made of elastomeric material.

Each of the first and second nonwoven webs may have a fiber composition selected from a group consisting of the following:

(a) 100% polypropylene staple fibers;
(b) 100% polyester staple fibers;
(c) 100% polyethylene staple fibers;
(d) 100% polyamide staple fibers;
(e) mixtures of polypropylene and rayon staple fibers having no more than 60% rayon; and
(f) 100% polyethylene/polypropylene bicomponent fibers.

The first and second nonwoven webs are preferably ultrasonically bonded at a plurality of spots having a total bond area in the range of 0.75% to 25%.

The invention also extends to a laminated fabric comprising first and second apertured films made of inelastic material ultrasonically bonded directly to each other at spaced intervals through holes in an elastomeric scrim sandwiched therebetween.

The invention further extends to a laminated fabric comprising first and second nonwoven webs of inelastic material ultrasonically bonded to each other at spaced intervals with an elastomeric film sandwiched therebetween, wherein each of said first and second nonwoven webs has a fiber composition selected from a group consisting of the following:

(a) 100% polypropylene staple fibers;
(b) 100% polyester staple fibers;
(c) 100% polyethylene staple fibers;
(d) 100% polyamide staple fibers;
(e) mixtures of polypropylene and rayon staple fibers having no more than 60% rayon; and
(f) 100% polyethylene/polypropylene bicomponent fibers.

Each of the said first and second nonwoven webs preferably comprise carded and thermally bonded fibers.

The invention also extends to a method for manufacturing a laminated fabric which can be elastically stretched in a machine direction, comprising the steps of:

stretching a liquid-permeable web of elastomeric material on said machine direction;

sandwiching said liquid-permeable web of elastomeric material between first and second nonwoven webs; and

ultrasonically bonding said first and second nonwoven webs to each other at a plurality of spaced bonding points, whereby said liquid-permeable web of elastomeric material occupies interstices between said spaced bonding points.

The web of elastomeric material is preferably a scrim.

The scrim may have a first mesh in a machine direction which is greater than a second mesh in a cross direction.

Alternatively the scrim may have a first mesh in a machine direction which is equal to a second mesh in a cross direction.

As a further possibility the scrim may have a plurality of mutually parallel machine direction strands and a plurality of mutually parallel cross direction strands, said machine direction strands and said cross direction strands being fused at their crossover points.

The scrim may comprise a plurality of interconnected strands made of elastomeric material.

The first and second nonwoven webs preferably have a fiber composition selected from a group consisting of the following:
(a) 100% polypropylene staple fibers;
(b) 100% polyester staple fibers;
(c) 100% polyethylene staple fibers;
(d) 100% polyamide staple fibers;
(e) mixtures of polypropylene and rayon staple fibers having no more than 60% rayon; and
(f) 100% polyethylene/polypropylene bicomponent fibers.

The first and second nonwoven webs are preferably ultrasonically bonded at a plurality of spots having a total bond area in the range of 0.75% to 25%.

The web of elastomeric material may be a film.

## Claims

1. A laminated fabric comprising first and second nonwoven webs or apertured films of inelastic material ultrasonically bonded to each other at spaced intervals through holes in an elastomeric web sandwiched therebetween.

2. The laminated fabric claimed in claim 1 wherein the elastomeric web is a scrim.

3. The laminated fabric as claimed in claim 1 or claim 2 wherein each of said first and second nonwoven webs comprises thermally bonded fibres.

4. The laminated fabric as claimed in claim 1 or claim 2 wherein each of said first and second nonwoven webs comprises spunbond fibres.

5. The laminated fabric as claimed in any one of claims 1 to 4 wherein said scrim has a first mesh in a machine direction which is greater than a second mesh in a cross direcrtion.

6. The laminated fabric as claimed in any one of claims 1 to 4 wherein said scrim has a first mesh in a machine direction which is equal to a second mesh in a cross direction.

7. The laminated fabric as claimed in any one of claims 1 to 4 wherein said scrim has a plurality of mutually parallel machine direction strands and a plurality of mutually parallel cross direction strands, said machine direction strands and said cross direction strands being fused at their crossover points.

8. The laminated fabric as claimed in any one of claims 1 to 7 wherein said scrim comprises a plurality of interconnected strands made of elastomeric material.

9. The laminated fabric as claimed in any one of claims 1 to 8 wherein each of said first and second nonwoven webs has a fiber composition selected from a group consisting of the following:
(a) 100% polypropylene staple fibers;
(b) 100% polyester staple fibers;
(c) 100% polyethylene staple fibers;
(d) 100% polyamide staple fibers;
(e) mixtures of polypropylene and rayon staple fibers having no more than 60% rayon; and
(f) 100% polyethylene/polypropylene bicomponent fibers.

10. The laminated fabric as claimed in any one of claims 1 to 9 wherein the elastomeric web is a film.

11. The laminated fabric as claimed in any one of the preceding claims wherein the first and second nonwoven webs or apertured films made of inelastic material are bonded directly to each other.

12. The laminated fabric as claimed in any one of claims 1 to 10 wherein each of said first and second nonwoven webs comprises carded and thermally bonded fibers.

13. The laminated fabric as claimed in any one of claims 1 to 12 wherein said first and second nonwoven webs or apertured films are ultrasonically bonded at a plurality of spots having a total bond area in the range of 0.75% to 25%.

14. A method for manufacturing a laminated fabric which can be elastically stretched in a machine direction, comprising the steps of:
   stretching a liquid-permeable web of elastomeric material on said machine direction;
   sandwiching said liquid-permeable web of elastomeric material between first and second nonwoven webs or apertured films; and
   ultrasonically bonding said first and second nonwoven webs or apertured films to each other at a plurality of spaced bonding points, whereby said liquid-permeable web of elastomeric material occupies interstices between said spaced bonding points.

15. The method as claimed in claim 14, wherein said web of elastomeric material is a scrim.

16. The method as claimed in claim 15, wherein said scrim has a first mesh in a machine direction which is greater than a second mesh in a cross direction.

17. The method as claimed in claim 15, wherein said scrim has a first mesh in a machine direction which is equal to a second mesh in a cross direction.

18. The method as claimed in claim 15, wherein said scrim has a plurality of mutually parallel machine firection strands and a plurality of mutually parallel cross direction strands, said machine direction strands and said cross direction strands being fused at their crossover points.

19. The method as claimed in claim 15, wherein said scrim comprises a plurality of interconnected strands made of elastomeric material.

20. The method as claimed in any one of claims 14 to 19, wherein each of said first and second nonwoven webs has a fiber composition selected from a group consisting of the following:
   (a) 100% polypropylene staple fibers;
   (b) 100% polyester staple fibers;
   (c) 100% polyethylene staple fibers;
   (d) 100% polyamide staple fibers;
   (e) mixtures of polypropylene and rayon staple fibers having no more than 60% rayon; and
   (f) 100% polyethylene/polypropylene bicomponent fibers.

21. The method as claimed in any one of claims 14 to 20, wherein said first and second nonwoven webs or apertured films are ultrasonically bonded at a plurality of spots having a total bond area in the range of 0.75% to 25%.

22. The method as claimed in any one of claims 14 to 21, wherein said web of elastomeric material is a film.

FIG. 1

FIG. 2